# EUROPEAN PATENT SPECIFICATION

(11) **EP 1 168 992 B1**
(45) Date of publication and mention of the grant of the patent: **28.12.2005**
(21) Application number: 00926817.8
(22) Date of filing: 06.04.2000
(51) Int. Cl.: A61F 5/445, A61F 5/448

(54) **SYSTEM FOR SURGICAL COLOSTOMY IMPLANTS**
SYSTEM FÜR CHIRURGISCHES KOLOSTOMIEIMPLANTAT
SYSTEME D'IMPLANTS CHIRURGICAUX POUR COLOSTOMIE

(30) Priority: 16.04.1999 IT TO990306
(43) Date of publication of application: 09.01.2002
(73) Proprietor: Bellini, Alberto, 10131 Torino (IT); Zanon, Claudio P. P., 10131 Torino (IT)
(72) Inventor: ZANON, Claudio, Pier, Paolo, I-10131 Torino (IT)
(74) Representative: Buzzi, Franco
(86) International application number: PCT/EP2000/003056
(87) International publication number: WO 2000/062722

(56) References cited:
- EP-A- 0 592 131
- WO-A-98/58691
- FR-A- 2 511 240
- GB-A- 2 045 084
- US-A- 4 183 357
- US-A- 5 766 249

## Description

The present invention relates in general to systems for surgical enterostomy, especially colostomy and ileostomy, implants. Such a system is known from WO-A-98/58691.

Further systems are known from, for example, international patent application WO87/03192 and US patent 4, 183, 357. In particular, the system disclosed in the second document comprises a cylindrical tubular part of essentially rigid biocompatible non-absorbent and non-biodegradable material suitable for application and fixing to an abdominal outlet orifice of the intestine, the system being such as to allow, on the one hand, the attachment and vascularization of the end of the intestine, and, on the other hand, the fitting of a bag, plug or the like.

Such systems have many disadvantages both in relation to their fitting, and to the effects of their use by the patient with particular reference to problems of hermetic sealing of the bag or plug or the like fitted to the tubular part, in addition to risks of stenosis and prolapse of the intestine. This explains why these systems appear not to have been taken up in practice.

The object of this invention is to overcome the above disadvantages, and to provide a system for surgical colostomy implants of the kind defined above that will be highly practical and effective both as patient.

According to the invention this object is achieved essentially owing to the fact that the wall of the tubular part comprises:
- a first axial portion whose outer and inner surfaces are each provided with an annular covering of porous material, over the outside and inside of which the end of the intestine can be fitted, its seromuscular and mucous layers being detached from each other and placed in intimate contact with the said coverings of porous material,
- a second axial portion suitable for the disengageable leakproof connection of the said bag, plug or the like, and
- an external annular flange that separates the said first and second axial portions whereby said first axial portion has a constant cross section.

The abovementioned coverings of porous material are gauze-like in structure and are conveniently made from a biocompatible material such as PROLENE®, DACRON® etc.

The invention will now be described in detail with reference to the accompanying drawings, provided purely by way of non-restrictive example, in which:
Figure 1 is a diagrammatic perspective view of a system for surgical colostomy implants according to the invention,
Figure 2 is an axial cross section on II-II as marked in Figure 1, and
Figure 3 is an exploded perspective view of Figure 2.

Referring to the drawings, a system for surgical colostomy implants according to the invention basically comprises a cylindrical tubular part 1 of essentially rigid biocompatible non-absorbent and non-biodegradable material. The tubular part 1 is normally made of a moulded plastic such as polyurethane, silicone or similar material.

This tubular part 1 has a circular cross section of approximately constant diameter, and its wall includes a first portion or axially inner portion 2, a second portion or axially outer portion 3, and an external radial annular flange 4 that separates the first axial portion 2 from the second 3.

The axial portion 2 is of greater length than the axial portion 3, and is designed to permit biological attachment and vascularization of the end of the intestine I where it meets an orifice A prepared in the patient's abdomen.

It is a fundamental feature of the invention that the outer and inner surfaces of the axial portion 2 are each provided with an annular covering of porous material, marked 5, 6 respectively. These annular coverings 5, 6 are conveniently - but not necessarily - composed of two folded portions of a single flexible body like a sock 7, and are gauze-like in structure. The coverings 5, 6 are in practice made from a thin net of biocompatible plastic material, preferably a commercially available material called PROLENE® or DACRON® etc.

As can clearly be seen in the drawings, the sock-like body 7 is inserted in such a way as to straddle over the free edge of the axial portion 2 of the tubular body 1, in such a way that the gauze-like coverings 5, 6 are in contact with the outer and inner surfaces of the axial portion 2 and terminate against the external annular flange 4.

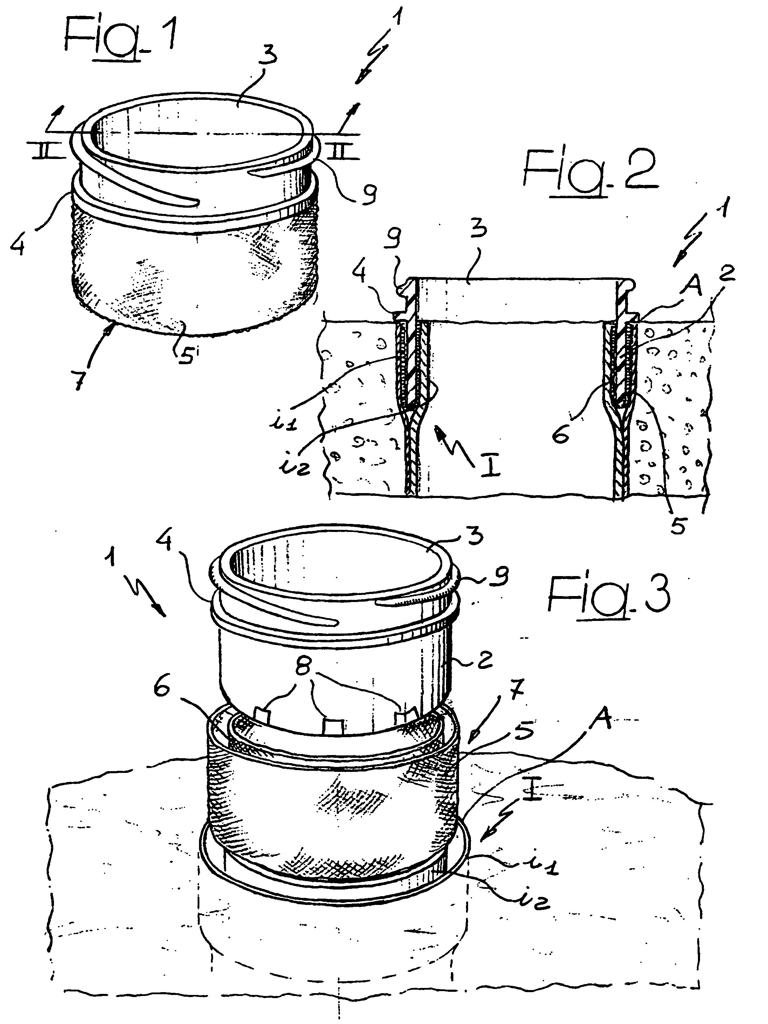

The wall of the first portion 2 of the tubular body 1 may also be provided with orifices or holes, such as those marked 8 in Figure 3, while the second axial portion 3 is formed with engagement members to allow it to be coupled in the manner of a connector to a bag, plug or the like. In the case of the example illustrated the engagement members, which have the general reference 9, are of the threaded sort: alternatively, they could take the form of bayonet, Luer lock or similar engagement members. Whichever form is adopted, the arrangement will be such that, in the connected condition, the annular flange 4 acts as an end stop so that the bag, plug or the like is hermetically sealed.

For the surgical implantation of the system described above, the procedure is as follows: the abdominal orifice A having been prepared, the intestine I is sutured, in the vicinity of its free end, to the edge of the abdominal orifice A, in such a way that this end projects to a greater or lesser degree from the orifice A. The seromuscular layer i₁ and the mucous layer i₂ of the end of the intestine I are then detached circumferentially from each other and the axial portion 2 of the tubular part 1 is inserted into the resulting annular interstice in the end of the intestine I, until the external annular flange 2 meets the intestine.

In this way the layers i₁, i₂ of the intestine I are placed in.intimate circumferential contact with the gauze-like coverings 5, 6 and can therefore be sutured to them. This intimate contact provides an effective and leakproof vascularized biological attachment between the end of the intestine I and the tubular part 1. This attachment can be further improved by the presence of the optional orifices 8.

In this way the tubular part 1 is thus efficiently secured to the abdomen of the patient, normally without the need for any additional means of holding it in place.

Needless to say, the details of construction and the embodiments can vary considerably from those described and illustrated, without thereby departing from the scope of the present invention as defined in the following claims. For example, in a variant that is not illustrated, the cylindrical tubular part 1 could be fitted with a pressure-relief valve unit.

## Claims

1. A system for surgical colostomy and ileostomy implants, comprising a cylindrical tubular part (1) of essentially rigid biocompatible non-absorbent and non-biodegradable material suitable for application and fixing to an abdominal outlet orifice (A) of the intestine (I), the system being such as to allow, on the one hand, the attachment and vascularization of the end of the intestine (I), and, on the other hand, the fitting of a bag, plug or the like, the wall of the said cylindrical tubular part (1) comprising:
- a first axial portion (2) whose outer and inner surfaces are each provided with respective annular coverings of porous material (5, 6), and
- a second axial portion (3) designed for the disengageable leakproof connection of the said bag, plug or the like,
which system is **characterized in that**:
- said first axial portion (2) has a constant cross section,
- an external radial annular flange (4) separates the said first and second axial portions (2, 3),
whereby the end of the intestine (I) can be fitted over the outside and inside of said first axial portion (2) with its seromuscular (i₁) and mucous (i₂) layers detached from each other and placed in intimate contact with the said coverings of porous material (5, 6) until meeting said annular flange (4).

2. System according to Claim 1, **characterized in that** the said annular coverings of porous material (5, 6) are gauze-like in structure.

3. System according to Claim 1 or Claim 2, **characterized in that** the said annular coverings of porous material (5, 6) are formed by two folded portions of a single flexible body (7).

4. System according to any one of Claims 1 to 3, **characterized in that** the said annular coverings of porous material (5, 6) are a biocompatible plastic material.

5. System according to any one of the preceding claims, **characterized in that** the said first axial portion (2) of the wall of the tubular part (1) is perforated.

6. System according to one or more of the preceding claims, **characterized in that** the said second portion (2) of the wall of the tubular part (1) is formed with threaded (9), bayonet, Luer lock or similar engagement members.

7. System according to any one of the preceding claims, **characterized in that** the said cylindrical tubular part (1) is of circular section with an approximately constant diameter.

## Patentansprüche

1. System für chirurgische Kolostomie- und Ileosomie-Implantate, aufweisend einen zylindrischen, röhrenförmigen Teil (1) eines im Wesentlichen steifen, bio-kompatiblen, nicht-absorbierenden und nicht biologisch abbaubaren Materials, das geeignet ist für die Anwendung auf und die Befestigung an einer Unterleibs-Ausgangs-Öffnung (A) des Darms (I), wobei das System derart ist, um auf der einen Seite die Befestigung und Vaskularisierung des Ende des Darmes (I) und auf der anderen Seite den Einbau eines Beutels, eines Stopfens oder ähnlichem zu erlauben, wobei die Wand des zylindrischen, röhrenförmigen Teils (1) aufweist:
- einen ersten axialen Abschnitt (2), dessen äußere und innere Oberfläche jede mit jeweiligen ringförmigen Überzügen eines porösen Materials (5, 6) versehen sind, und
- ein zweiter axialer Abschnitt (3), welcher für die entfernbare, lecksichere Verbindung des Beutels, des Stopfens oder ähnlichem konstruiert ist,
welches System **dadurch gekennzeichnet ist, dass**:
- der erste axiale Abschnitt (2) einen konstanten Querschnitt aufweist,
- ein äußerer, radialer, ringförmiger Flansch (4) den ersten axialen Abschnitt (2) und den zweiten axialen Abschnitt (3) voneinander trennt,
wobei das Ende des Darms (I) über die Außenseite und Innenseite des ersten axialen Abschnitts (2) eingesetzt werden kann, mit seinen seromuskularen (i₁) und mukösen (i₂) Schichten voneinander abgetrennt und in engern Kontakt mit den Überzügen von porösem Material (5, 6) gesetzt, bis zum Auftreffen auf den ringförmigen Flansch (4).

2. System nach Anspruch 1, **dadurch gekennzeichnet, dass** die ringförmigen Überzüge eines porösen Materials (5, 6) in ihrer Struktur Gaze-artig sind.

3. System nach Anspruch 1 oder Anspruch 2, **dadurch gekennzeichnet, dass** die ringförmigen Überzüge von porösem Material (5, 6) durch zwei gefaltete Abschnitte eines einzigen, flexiblen Körpers (7) gebildet sind.

4. System nach irgendeinem der Ansprüche 1 bis 3, **dadurch gekennzeichnet, dass** die ringförmigen Überzüge von porösem Material (5, 6) ein biokompatibles Kunststoffmaterial sind.

5. System nach irgendeinem der vorangegangenen Ansprüche, **dadurch gekennzeichnet, dass** der erste axiale Abschnitt (2) der Wand des röhrenförmigen Teils (1) perforiert ist.

6. System nach einem oder mehreren der vorangegangenen Ansprüche, **dadurch gekennzeichnet, dass** der zweite Abschnitt (2) der Wand des röhrenförmigen Teils (1) mit einem mit einem Gewinde versehenen (9) Bajonett, Luer-Schloß oder ähnlichen Eingriffselementen gebildet ist.

7. System nach irgendeinem der vorangegangenen Ansprüche, **dadurch gekennzeichnet, dass** der zylindrische, röhrenförmige Teil (1) von einem kreisförmigen Querschnitt mit einem annähernd konstanten Durchmesser ist.

## Revendications

1. Système pour implants chirurgicaux d'iléostomie et de colostomie, comprenant une pièce tubulaire cylindrique (1) d'un matériau essentiellement rigide, biocompatible, non-absorbant et non-biodégradable adapté pour l'application et la fixation sur un orifice de sortie abdominale (A) de l'intestin (I), le système permettant d'une part, la fixation et la vascularisation de l'extrémité de l'intestin (I) et d'autre part, la mise en place d'une poche, d'un embout ou similaire, la paroi de ladite pièce tubulaire cylindrique (1) comprenant :
- une première partie axiale (2) dont les surfaces interne et externe sont chacune munies de revêtements annulaires respectifs en matériau poreux (5, 6) et
- une seconde partie axiale (3) conçue pour le raccord détachable anti-fuites de ladite poche, dudit embout ou similaire,
lequel système est **caractérisé en ce que** :
- ladite première partie axiale (2) a une section transversale constante,
- une bride annulaire et radiale externe (4) sépare lesdites première et seconde parties axiales (2, 3),
moyennant quoi l'extrémité de l'intestin (I) peut être placée sur l'extérieur et l'intérieur de ladite première partie axiale (2) avec ses couches séromusculaire (i₁) et muqueuse (i₂) séparées l'une de l'autre et placées en contact étroit avec lesdits revêtements en matériau poreux (5, 6) jusqu'à rencontrer ladite bride annulaire (4).

2. Système selon la revendication 1, **caractérisé en ce que** lesdits revêtements annulaires en matériau poreux (5, 6) ont une structure rappelant la gaze.

3. Système selon la revendication 1 ou la revendication 2, **caractérisé en ce que** lesdits revêtements annulaires en matériau poreux (5, 6) sont formés par deux parties pliées d'un seul corps flexible (7).

4. Système selon l'une quelconque des revendications 1 à 3, **caractérisé en ce que** lesdits revêtements annulaires en matériau poreux (5, 6) sont un matériau plastique biocompatible.

5. Système selon l'une quelconque des revendications précédentes, **caractérisé en ce que** ladite première partie axiale (2) de la paroi de la pièce tubulaire (1) est perforée.

6. Système selon l'une ou plusieurs des revendications précédentes, **caractérisé en ce que** ladite seconde partie (2) de la paroi de la pièce tubulaire (1) est formée par des éléments filetés (9), une baïonnette, un embout Luer ou des éléments d'engagement similaires.

7. Système selon l'une quelconque des revendications précédentes, **caractérisé en ce que** ladite pièce tubulaire cylindrique (1) est de section circulaire avec un diamètre approximativement constant.
